# EUROPEAN PATENT SPECIFICATION

(11) **EP 0 851 893 B1**
(45) Date of publication and mention of the grant of the patent: **05.07.2000**
(21) Application number: 96931620.7
(22) Date of filing: 17.09.1996
(51) Int. Cl.: C08K 5/3492, C08K 5/3445, C07D 251/64, C07D 251/18, C07D 487/04

(54) **FORMALDEHYDE-FREE CROSSLINKERS**
FORMALDEHYDFREIE VERNETZER
AGENTS RETICULANTS EXEMPTS DE FORMALDEHYDE

(30) Priority: 20.09.1995 US 4055
(43) Date of publication of application: 08.07.1998
(73) Proprietor: Cytec Technology Corp., Wilmington, Delaware 19801 (US)
(72) Inventor: ZHAO, Hong, Stamford, CT 06902 (US); LAWLESS, Barry, A., Milford, CT 06460 (US); LEES, Robert, G., Stamford, CT 06902 (US)
(74) Representative: Diehl, Hermann, Dr. Dipl.-Phys.
(86) International application number: US9614918
(87) International publication number: WO9711119

(56) References cited:
- EP-A- 0 698 627
- WO-A-96/17879

## Description

### BACKGROUND OF THE INVENTION

### Field of the invention

This invention relates to the N-1,2,2-trihydrocarbyloxyethyl derivatives of certain amino compounds, methods for preparing such derivatives, and compositions containing such derivatives. These derivatives and compositions are particularly suitable for use as crosslinking agents in curable compositions such as coatings, and do not release formaldehyde as a volatile by-product when cured.

### Description of Related Art

Various derivatives of amino compounds, such as amino-1,3,5-triazines and glycolurils, are described in the literature for use in a wide variety of fields. Certain of these derivatives, such as the partially or fully alkoxymethylated derivatives of melamine, guanamines and glycoluril, are useful as crosslinkers in curable compositions which contain resins having active hydrogen groups. See, for example, US4064191, US4081426, US4101520, US4118437, US4129681, US4243705, US4271277, US4276212, US4330458, US4374164, US4433143, US4425466, US4873298, US5155201 and US5256713.

While these alkoxymethylated derivatives provide excellent results in a number of aspects, they also have the disadvantage of releasing formaldehyde as a volatile by-product under curing conditions. Despite the excellent films which can be achieved with these systems, the coatings industry is under pressure to reduce the environmentally undesirable emission of formaldehyde. As a result, it has long been a desire of industry to find acceptable alternative crosslinkers which do not emit formaldehyde upon cure.

One such non-formaldehyde emitting alternative is the class of isocyanate and carbamate functional 1,3,5-triazine crosslinking agents disclosed in commonly owned US4939213, US5084541, US5288865, EP-A-0604922 (corresponding to United States Application Serial No. 07/998,313, filed December 29, 1992), EP-A-0624577 (corresponding to United States Application Serial No. 08/061,905, filed May 14, 1993), EP-A-0649842 (corresponding to United States Application Serial No. 08/138,581, filed October 15, 1993), W095/30663 (corresponding to United States Application Serial No. 08/239,009, filed May 6, 1994), WO96/04258 (corresponding to United States Application Serial No. 08/286,835, filed August 5,1994), WO96/11915 (corresponding to United States Application Serial No. 08/324,549, filed October 18, 1994) and WO96/15185 (corresponding to United States Application Serial No. 08/340,950, filed November 16, 1994). Other non-formaldehyde emitting alternatives include, for example, the class of lactam substituted 1,3,5-triazine crosslinking agents disclosed in commonly owned WO93/10117 (corresponding to United States Application Serial No. 07/973,676, filed November 9, 1992), and the class of acetal and enamine functional 1,3,5-triazine crosslinking agents disclosed in commonly owned WO96/XXXXX (corresponding to United States Application Serial No. 08/408,323, filed March 21, 1995). WO 96/17879 discloses a resin composition useful as a binder comprising the reaction product of an amine derivative chosen from melamine, glycolurile or their mixtures with a C₁ to C₈ dialkoxyethanal. The reaction mixture is then mixed, preferably reacted with a polyol having 2 or more hydroxyl groups.

The aforementioned have been found to be particularly useful as crosslinkers in coating compositions based on active hydrogen and/or epoxy groups containing resins, with the cured coatings possessing a wide range of desirable properties.

While some of these alternatives have shown great promise, the search continues for replacements for traditional amino derivative crosslinkers, which replacements retain many of the desirable properties of the traditional crosslinkers but which emit little or no formaldehyde on cure.

### SUMMARY OF THE INVENTION

We have now discovered a new class of amino compound derivatives prepared without formaldehyde, which is capable of functioning as a highly compatible crosslinking agent for the wide variety of functional materials useable in traditional amine-formaldehyde crosslinked systems. Curable systems (such as coatings) can be formulated with these new amino compound derivatives to have no formaldehyde release on cure, with the resulting crosslinked articles (such as crosslinked films) possessing physical and appearance characteristics comparable to crosslinked articles derived from curable systems based on traditional amine-formaldehyde crosslinkers.

In its overall concept, the present invention is an N-1,2,2-trihydrocarbyloxyethyl derivative of an amino compound, comprising the reaction product of:
(i) an amino compound having at least two =NH groups, selected from the group consisting of amino-1,3,5-triazines, glycolurils and oligomers thereof,
(ii) a 2,2-dihydrocarbyloxy ethanal, and
(iii) a hydrocarbylol, excluding polyols having two or more hydroxyl groups, the reaction product containing, on average, at least 1.25 moles of combined 2,2-dihydrocarbyloxy ethanal per mole of amino compound, and at least about 2.0 1,2,2-trihydrocarbyloxyethyl groups per molecule of derivative.

The present invention also includes a process of preparing such N-1,2,2-trihydrocarbyloxyethyl derivative of an amino compound, comprising the steps of reacting (i), (ii) and (iii) under conditions so as to result in a derivative containing, on average, at least 1.25 moles of combined 2,2-dihydrocarbyloxy ethanal per mole of amino compound, and at least about 2.0 1,2,2-trihydrocarbyloxyethyl groups per molecule of derivative.

Depending on the types and proportions of starting components and other reaction conditions as described in further detail below, the derivatives in accordance with the present invention may comprise substantially a single species of monomeric compound, or may comprise a complex mixture of monomeric and oligomeric compounds. The monomeric compounds also form a specific part of the present invention, and can generally be described as compounds comprising an amino core having pendant therefrom at least two 1,2,2-trihydrocarbyloxyethyl groups, of the following general formula (I) or (II): 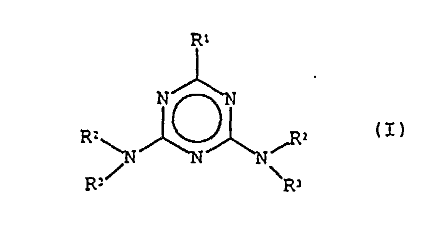 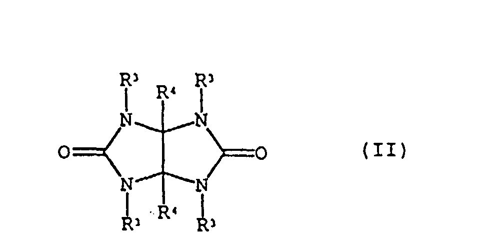 wherein
- R¹: is selected from H, a hydrocarbyl and -N(R²)(R³);
- each R²: is independently selected from H and a hydrocarbyl;
- each R³: is independently selected from H, a hydrocarbyl and an R group;
- each R⁴: is independently selected from H and a hydrocarbyl; and
- each R: group is independently a group of the general formula (III) 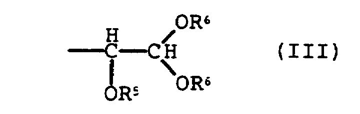
wherein
- each R⁵: is independently selected from H and a hydrocarbyl, and
- each R⁶: is independently a hydrocarbyl, or together form a hydrocarbylene bridge;
with the proviso that, per molecule, at least two of the R³ groups are independently an R group, and at least two R⁵ groups are independently a hydrocarbyl.

In any event, the aforementioned compounds and derivatives must contain, on average, at least about 2.0 1,2,2-trihydrocarbyloxyethyl groups (e.g., a group of the formula (III), wherein R⁵ is a hydrocarbyl group) per molecule, which makes these compositions particularly suitable for use as crosslinking agents in a variety of end applications. The present invention, consequently, also relates to curable compositions comprising (a) a crosslinker component comprising the compounds and/or compositions in accordance with the present invention, and (b) a resin component comprising a compound containing at least two groups capable of reacting with the 1,2,2-trihydrocarbyloxyethyl groups of (a).

Without wishing to be bound by any particular theory, it is believed that the derivatives in accordance with the present invention are primarily reactive via the activated ether of the 1,2,2-trihydrocarbyloxyethyl group (e.g., the hydrocarbyloxy in the 1 position). It has been surprisingly found that, without the presence of sufficient such activated ether functionality, good curing results cannot be obtained. Groups capable of reacting with 1,2,2-trihydrocarbyloxyethyl groups are, therefore, groups capable of reacting with activated ether groups within the meaning of the present invention, which are the same types of groups that are reactive with the alkoxymethyl and methylol functionality of traditional amine-formaldehyde crosslinkers.

A particularly advantageous such use of the curable compositions of the present invention is in the form of a coating composition. The present invention, therefore, also relates to curable coating compositions, methods for coating substrates as well as the substrates coated therewith, crosslinked films or objects derived from the curable composition, and various other end uses thereof.

As indicated above, the compounds and compositions of the present invention are prepared without using formaldehyde and therefore are formaldehyde-free. Curable compositions employing these compounds and compositions as crosslinkers can also be formulated as formaldehyde free systems. Other advantages include rapid cure, adaptability to waterbome coatings systems, and ability to produce fully cured coatings which have excellent appearance and film and resistance properties.

These and other features and advantages of the present invention shall be more readily understood by those of ordinary skill in the art from a reading of the following detailed description.

### DETAILED DESCRIPTION OF THE PREFERRED EMBODIMENTS

### N-1,2,2-Trihydrocarbyloxyethyl Derivatives

As indicated above, the present invention is broadly an N-1,2,2-trihydrocarbyloxyethyl derivative of an amino compound, comprising the reaction product of:
(i) an amino compound having at least two =NH groups, selected from the group consisting of amino-1,3,5-triazines, glycolurils and oligomers thereof,
(ii) a 2,2-dihydrocarbyloxy ethanal, and
(iii) a hydrocarbylol, excluding polyols having two or more hydroxyl groups the reaction product containing, on average, at least about 1.25 moles of combined 2,2-dihydrocarbyloxy ethanal per mole of amino compound, and at least about 2.0 1,2,2-trihydrocarbyloxyethyl groups per molecule of derivative.

It should be noted that the term "hydrocarbyl," within the context of the present invention, is a group which contains carbon and hydrogen atoms and includes, for example, alkyl, aryl, aralkyl, alkenyl and substituted derivatives thereof.

Preferred as amino compounds are amino-1,3,5-triazines and glycolurils of the general formulas (IV) and (V): 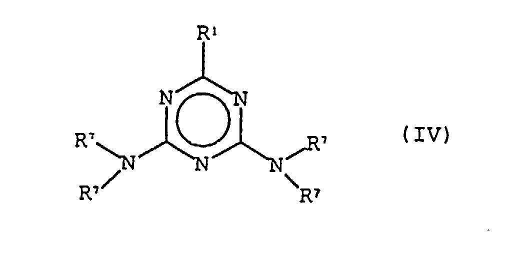 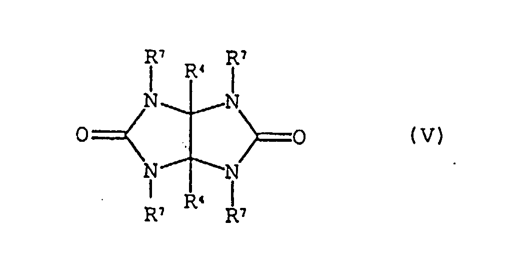 wherein
- R¹: is selected from H, a hydrocarbyl and -N(R⁷)₂,
- each R⁷: is independently selected from H and a hydrocarbyl, with the proviso that at least two groups R⁷ are H, and preferably that all R⁷ groups are H, and
- each R⁴: is independently selected from H and a hydrocarbyl, and
preferably that all R⁴ groups are H.

As specific examples of preferred amino compounds of the general formula (IV) (wherein all R⁷ groups are H) may be mentioned the guanamines, wherein R¹ is selected from H and a hydrocarbyl; more preferably H, an alkyl of 1 to 20 carbons atoms, an aryl of 6 to 20 carbon atoms and an aralkyl of 7 to 20 carbon atoms; and particularly a phenyl group (benzoguanamine), a methyl group (acetoguanamine) and a cyclohexyl group (cyclohexylcarboguanamine).

Another specific example of a preferred amino compound of the general formula (IV) (wherein all R⁷ groups are H) is melamine, wherein R¹ is -N(R⁷)₂.

The preferred amino compound of the general formula (V) (wherein all R⁴ and R⁷ groups are H) is glycoluril.

Preferred for the 2,2-dihydrocarbyloxy ethanal are compounds of the general formula (VI): 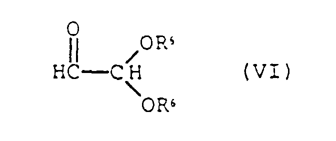 wherein
- each R⁶: is independently a hydrocarbyl, or together form a hydrocarbylene bridge.
Such 2,2-dihydrocarbyloxy ethanals and methods for their preparation are disclosed in US4835320. Preferred are those wherein each R⁶ is independently an alkyl of 1 to 8 carbon atoms or an alkenyl of 1 to 8 carbon atoms, as well as those wherein both R⁶ groups together form an alkylene bridge of 1 to 8 carbon atoms. Particularly preferred are those wherein each R⁶ is independently an alkyl of 1 to 8 carbon atoms, and those wherein both R⁶ groups together form an alkylene bridge of 1 to 4 carbon atoms. Specifically preferred examples include 2,2-dimethoxy ethanal, 2,2-diethoxy ethanal, 2-methoxy-2-ethoxy ethanal, 2,2-dipropoxy ethanal, 2,2-dibutoxy ethanal, 2,2-dipentoxy ethanal, 2,2-dihexoxy ethanal, 2,2-dicyclohexoxy ethanal, 2,2-ethylenedioxy ethanal and 2,2-propylenedioxy ethanal. Most preferred are 2,2-dimethoxy ethanal and 2,2-dibutoxy ethanal, and particularly 2,2-dimethoxy ethanal (DME).

Preferred for the hydrocarbylol are hydroxy group-containing compounds having from 1 to 20 carbon atoms such as, for example, alkylols, alkenols, phenols and alkoxyalkylols and excluding polyols having two or more hydroxyl groups.

As specific preferred examples thereof may be mentioned methanol, ethanol, propanols, butanols, ethylhexanols, allyl alcohol and phenol. Especially preferred are the alkylols of 1-8 carbon atoms, particularly methanol and butanols.

As indicated above, the derivatives in accordance with the present invention contain, on average, at least about 1.25 moles of combined 2,2-dihydrocarbyloxy ethanal per mole of amino compound, and at least about 2.0 1,2,2-trihydrocarbyloxyethyl groups per molecule of derivative.

The guanamine derivatives preferably contain on average from about 1.5 to about 2.0 moles of combined 2,2-dihydrocarbyloxy ethanal per mole of guanamine. A specific preferred embodiment is a substantially monomeric guanamine derivative which contains about 2.0 moles of combined 2,2-dihydrocarbyloxy ethanal per mole of guanamine. Such a substantially monomeric guanamine derivative is depicted by the general formula (I), wherein:
- R¹: is selected from H and a hydrocarbyl,
preferably selected from H, an alkyl of 1 to 20 carbons atoms, an aryl of 6 to 20 carbon atoms and an aralkyl of 7 to 20 carbon atoms, and
especially selected from a phenyl group, a methyl group and a cyclohexyl group;
each R² is selected from H and a hydrocarbyl, and
preferably H;
each R³ is a group of the formula (III);
each R⁵ is independently a hydrocarbyl,
preferably independently a hydrocarbyl of 1 to 20 carbon atoms selected from alkyls, alkenyls, phenyls and alkoxyalkylyls, and
especially independently an alkyl of 1 to 8 carbon atoms; and
each R⁶ is independently a hydrocarbyl, or together form a hydrocarbylene bridge,
preferably independently selected from an alkyl of 1 to 8 carbon atoms and an alkenyl of 1 to 8 carbon atoms, or together form an alkylene bridge of 1 to 8 carbon atoms, and
especially independently an alkyl of 1 to 8 carbon atoms, or together form an alkylene bridge of 1 to 4 carbon atoms.

The melamine derivatives preferably contain on average from about 2.0 to about 3.0 moles and, more preferably, from about 2.3 to about 3.0 moles, of combined 2,2-dihydrocarbyloxy ethanal per mole of melamine. A specific preferred embodiment is a substantially monomeric melamine derivative which contains about 3.0 moles of combined 2,2-dihydrocarbyloxy ethanal per mole of melamine. Such a substantially monomeric melamine derivative is depicted by the general formula (I), wherein:
- R¹: is -N(R²)(R³);
- each R²: is selected from H and a hydrocarbyl, and
preferably H;
- each R³: is a group of the formula (III);
- at: least two of the R⁵ groups, and preferably each of the R⁵ groups, are independently a hydrocarbyl,
preferably independently a hydrocarbyl of 1 to 20 carbon atoms selected from alkyls, alkenyls, phenyls and alkoxyalkylyls, and
especially independently an alkyl of 1 to 8 carbon atoms; and
- each R⁶: is independently a hydrocarbyl, or together form a hydrocarbylene bridge,
preferably independently selected from an alkyl of 1 to 8 carbon atoms and an alkenyl of 1 to 8 carbon atoms, or together form an alkylene bridge of 1 to 8 carbon atoms, and
especially independently an alkyl of 1 to 8 carbon atoms, or together form an alkylene bridge of 1 to 4 carbon atoms.

The glycoluril derivatives preferably contain on average from about 2.0 to about 4.0 moles and, more preferably, from about 3.0 to about 4.0 moles, of combined 2,2-dihydrocarbyloxy ethanal per mole of glycoluril. A specific preferred embodiment is a substantially monomeric glycoluril derivative which contains about 4.0 moles of combined 2,2-dihydrocarbyloxy ethanal per mole of glycoluril. Such a substantially monomeric glycoluril derivative is depicted by the general formula (II), wherein:
- each R³: is a group of the formula (III);
- each R⁴: is independently selected from H and a hydrocarbyl, and
preferably H;
- at: least two of the R⁵ groups, preferably at least three of the R⁵ groups, and especially each of the R⁵ groups, are independently a hydrocarbyl,
preferably independently a hydrocarbyl of 1 to 20 carbon atoms selected from alkyls, alkenyls, phenyls and alkoxyalkylyls, and
especially independently an alkyl of 1 to 8 carbon atoms; and
- each R⁶: is independently a hydrocarbyl, or together form a hydrocarbylene bridge,
preferably independently selected from an alkyl of 1 to 8 carbon atoms and an alkenyl of 1 to 8 carbon atoms, or together form an alkylene bridge of 1 to 8 carbon atoms, and
especially independently an alkyl of 1 to 8 carbon atoms, or together form an alkylene bridge of 1 to 4 carbon atoms.

### Processes for Preparing the Derivatives

The N-1,2,2-trihydrocarbyloxyethyl derivatives described above may be prepared in accordance with the present invention by reacting (i), (ii) and (iii) under conditions sufficient to produce a derivative having, on average, at least 1.25 moles of combined 2,2-dihydrocarbyloxy ethanal per mole of amino compound, and at least about 2.0 1,2,2-trihydrocarbyloxyethyl groups per molecule of derivative.

In one specific embodiment of the present process, (i) and (ii) are contacted in a first step in the presence of a basic catalyst to produce a 1-hydroxy-2,2-dihydrocarbyloxyethyl derivative intermediate, which in a subsequent step is reacted with (iii) (e.g., alkylated) under acidic conditions (preferably in the presence of an acid catalyst). The first step is typically conducted in an aqueous medium, at temperatures generally ranging from ambient to about 120°C. Upon completion of the first step, the intermediate is isolated and added to an organic solvent for the second step. Advantageously, an excess of the hydrocarbylol (iii) may be used as the organic solvent. The reaction of the intermediate from the first step and (iii) is typically carried out under conditions and with acid catalysts similar to those involved in an alkylating step for traditional amine-formaldehyde resins, which conditions and catalysts are well-known to those of ordinary skill in the art.

In a second specific embodiment of the present process, (i), (ii) and (iii) are concurrently contacted in the presence of an acid catalyst to directly result in the 1,2,2-trihydrocarbyloxy derivative. As with the second step above, this reaction is typically carried out under conditions and with acid catalysts similar to those involved in an alkylating step for traditional amine-formaldehyde resins, which conditions and catalysts are well-known to those of ordinary skill in the art.

### Curable Compositions

As described generally above, the curable compositions in accordance with the present invention comprise:
(a) a crosslinker component comprising an N-1,2,2-trihydrocarbyloxyethyl derivative as described above, and
(b) a resin component comprising a compound containing at least two groups capable of reacting with the 1,2,2-trihydrocarbyloxyethyl groups of (a).

In addition to the N-1,2,2-trihydrocarbyloxyethyl derivative crosslinker described in detail above, the crosslinker component may optionally comprise a variety of additional ingredients. For example, the crosslinker component may optionally contain other crosslinking agents, referred to herein as "co-crosslinkers," which include, particularly, active-hydrogen and epoxy reactive crosslinking agents such as, for example, traditional amine-formaldehyde resins, blocked and/or unblocked polyfunctional isocyanates, and isocyanate and carbamate functional 1,3,5-triazine carbamate crosslinkers.

As suitable amine-formaldehyde resins may be mentioned the partially or substantially fully methylolated, partially or substantially fully etherified amino compounds based on melamine, guanamines, glycolurils and urea. In general, such amine-formaldehyde resins are well known to those of ordinary skill in the art (see, for example, the numerous previously incorporated references) and are generally available commercially. Most commonly, they include melamines, guanamines such as benzo-, aceto- and cyclohexylcarbo-guanamines, glycolurils and ureas, as well as the at least partially N-alkylolated and N-alkoxyalkylated derivatives thereof, and oligomers thereof.

As specific examples of commercially available amino resins of the type described above may be mentioned those sold under the trademarks CYMEL® and BEETLE® of Cytec Industries, Inc. (West Paterson, New Jersey).

Polyisocyanate crosslinking agents, including blocked forms thereof, are generally well known in the art and have been extensively used in coating compositions in a monomeric, oligomeric and/or polymeric form, and preferably contain at least two reactive isocyanate groups. As specific examples of such may be mentioned hexamethylene diisocyanate; 2,2,4-trimethylhexamethylene diisocyanate; 2,4,4-trimethylhexamethylene diisocyanate; meta-a,a,a',a'-tetramethylxylylenediisocyanate (commercially available under the trade designation m-TMXDI® aliphatic isocyanate from Cytec Industries Inc., West Paterson, New Jersey); para-α,α,α',α'-tetramethylxylylenediisocyanate (available under the trade designation p-TMXDI® aliphatic isocyanate from Cytec Industries Inc., West Paterson, New Jersey); 1-isocyanato-3,3,5-trimethyl-5-isocyanatomethyl cyclohexane (isophorone diisocyanate, abbreviated as IPDI); bis(4-isocyanatocyclohexyl)methane (hydrogenated MDI): biuret derivatives of various diisocyanates including, for example, hexamethylene diisocyanate (commercially available under the trade designation Desmodur® N of Miles Inc., Pittsburgh, Pennsylvania); uretdione derivatives of various diisocyanates including, for example, hexamethylene diisocyanate and IPDI; isocyanurate derivatives of various diisocyanates including, for example, hexamethylene diisocyanate (commercially available under the trade designation Desmodur® N 3390 of Miles Inc., Pittsburgh, Pennsylvania) and IPDI (commercially available under the trade designation IPDI® T 1890 polyisocyanate of Huls America, Inc., Piscataway, N.J.); and urethane adducts of diisocyanates with polyols such as, for example, ethylene glycol, propylene glycol, neopentyl glycol, trimethylolpropane, pentaerythritol and the like, as well as oligomeric and polymeric polyols, for example, the 3:1 meta-α,α,α',α'-tetramethylxylylenediisocyanate/trimethylolpropane adduct (commercially available under the trade designation CYTHANE® 3160 aliphatic polyisocyanate of Cytec Industries Inc., West Paterson, New Jersey), and the 3:1 IPDI/trimethylolpropane adduct (commercially available under the trade designation SPENLITE® P 25-A4-60 aliphatic urethane prepolymer of Reichhold Chemicals, Research Triangle Park, North Carolina).

The polyisocyanates may be blocked in a well-known manner with, for example, lower alkyl alcohols and oximes.

As suitable isocyanate and carbamate functional 1,3,5-triazine carbamate crosslinkers may be mentioned those having on average at least two isocyanate and/or carbamate groups attached to one or more 1,3,5-triazine cores. In general, these 1,3,5-triazine compounds are also well known to those of ordinary skill in the art, as exemplified by the numerous references previously incorporated above.

Suitable for use as the resin component are compounds containing at least two groups capable of reacting with the 1,2,2-trihydrocarbyloxyethyl groups of (a) such as, for example, active hydrogen and/or epoxy groups, which are generally the same types of materials suitable for use in traditional amine-formaldehyde resin crosslinked systems.

Preferred are the polyfunctional active hydrogen group containing compound. Active hydrogen-containing functionality, as utilized herein, refers to functional groups which contain active hydrogens as is generally well-known to those of ordinary skill in the art and includes, most commonly, hydroxyl, carboxyl and amino groups. When utilized herein, hydroxyl is preferred.

Suitable such polyfunctional hydroxy group containing materials are again generally well known to those skilled in the art, and contain at least two and preferably more than two hydroxy groups. Reference may be had to previously incorporated US4939213, US5084541, US5288865, EP-A-0604922 (corresponding to United States Application Serial No. 07/998,313, filed December 29, 1992), EP-A-0624577 (corresponding to United States Application Serial No. 08/061,905 filed May 14, 1993), EP-A-0649842 (corresponding to United States Application Serial No. 08/138,581, filed October15, 1993), W095/30663 (corresponding to United States Application Serial No. 08/239,009, filed May 6, 1994), W096/04258 (corresponding to United States Application Serial No. 08/286,835, filed August 5, 1994), WO96/11915 (corresponding to United States Application Serial No. 08/324,549, filed October 18, 1994) and WO96/15185 (corresponding to United States Application Serial No. 08/340,950, filed November 16, 1994) for further details.

As examples of preferred polyfunctional hydroxy group containing materials may be mentioned acrylic or polyester backbone resins. Illustrative examples include acrylic resins which may be obtained by the copolymerization of acrylic or methacrylic esters with hydroxyfunctional acrylic or methacrylic esters such as hydroxyethyl acrylate or methacrylate, optionally with simultaneous use of additional vinyl compounds such as, for example, styrene. Illustrative examples of the polyfunctional hydroxy group containing materials also include polyester resins which may be obtained, for example, by the reaction of polycarboxylic acids with excess quantities of polyhydric alcohols. Other suitable polyfunctional hydroxy group containing resins include polyurethane prepolymers, alkyds, as well as hydroxy group containing epoxy prepolymers such as those resulting from the reaction of a polyfunctional epoxy group containing compound with an amine or with a polyfunctional carboxylic acid derivative.

In general, such resins may have pendent or terminal hydroxyl functionalities and preferably have the following characteristics: weight average molecular weights (Mw) of from about 750 to about 7000, and more preferably from about 2000 to about 5000; and hydroxyl numbers of from about 20 to about 120 mg KOH/g resin.

For waterbome applications, polyfunctional hydroxy group containing materials having thereon aqueous dispersion promoting groups such as carboxylic or sulfonic functionalities and higher molecular weights are generally usable, such as disclosed in (WO96/15185, as well as GB1530022, EP-A-0568134, EP-A-0663413, US5075370 and US5342878. Solid polyfunctional hydroxy group containing materials are suitable for use in powder coatings. For solvent borne coatings, liquid polyfunctional hydroxy group containing materials are preferred. However, solid polyfunctional hydroxy group containing materials may be used in cases when the solids are soluble in the solvent used in a particular formulation. Specific suitable hydroxyl functional resins will be readily recognized by those of ordinary skill in the art depending upon the desired end use.

Commercially available examples of polyfunctional hydroxy group containing materials include JONCRYL® 500 acrylic resin, a product of S.C.Johnson & Sons, Racine, WI; ACRYLOID® AT-400 acrylic resin, a product of Rohm & Haas, Philadelphia, PA; CYPLEX® 1531 polyester resin, a product of Cytec Industries, West Paterson, NJ; CARGILL 3000 and 5776 polyester resins, products of Cargill, Minneapolis, MN; TONE® polyester resin, a product of Union Carbide, Danbury, CT; K-FLEX® XM-2302 and XM-2306 resins, products of King Industries, Norwalk, CT; CHEMPOL® 11-1369 resin, a product of Cook Composites and Polymers, Port Washington, WI; JONCRYL® 540 acrylic emulsion polymer, a product of S.C.Johnson & Sons, Racine, WI; RHOPLEX® AC-1024 acrylic emulsion resin, a product of Rohm & Haas, Philadelphia, PA; XC® 4005 water reducible acrylic resin, a product of Cytec Industries, West Paterson, NJ; CRYLCOAT® 3494 solid hydroxy terminated polyester resin, a product of UCB CHEMICALS USA, Smyma, GA; RUCOTE® 101 polyester resin, a product of Ruco Polymer, Hicksville, NY; JONCRYL® SCX-800-A and SCX-800-B hydroxyfunctional solid acrylic resins, products of S.C.Johnson & Sons, Racine, WI); and ALFTALAT® AN 745 hydroxyfunctional polyester resin, a product of Hoechst Corporation.

### Other Ingredients

In addition to the crosslinker and resin components described in detail above, the curable compositions of the present invention may optionally comprise a variety of additional ingredients normal for any particularly chosen end use.

One common such additional ingredient is a cure catalysts for increasing the cure rate and thereby reducing the cure temperature and/or cure time of the systems described herein. Suitable cure catalysts include those typically suited for use in traditional amine-formaldehyde crosslinked systems, such as protic acid catalysts and Lewis acid catalysts. As examples of the protic acid catalysts may be mentioned sulfonic acids such as p-toluene sulfonic acid or dodecyl benzene sulfonic acid. Other examples include aryl and alkyl acid-phosphates and pyrophosphates, carboxylic acids, sulfonimides and mineral acids. Latent acidic catalysts, such as amine-blocked p-toluene sulfonic acid or amine-blocked dodecyl benzene sulfonic acid, are included within the meaning of protic acid catalysts. As examples of the Lewis acid catalysts may be mentioned compounds of aluminum, boron, magnesium, antimony and tin. The use of cure catalysts are optional in the present systems and, when utilized, are generally added in amounts ranging from about 0.001 wt % to about 6.0 wt %, and preferably up to about 2.0 wt %, based on the combined weight of the resin and crosslinker components (total resin solids).

The present curable compositions may also contain a solvent of the type typically found in coatings applications including, for example, alcohols, ketones, esters, aliphatic hydrocarbons, aromatic hydrocarbons and halogenated hydrocarbons. In waterbome coating applications, the curable compositions may contain, in addition to water, a co-solvent and an aqueous dispersion promoting material such as ethylhexanol, Texanol® (a C8-hydroxyalkyl ester of methylpropionic acid commercially available from Eastman Chemical Company), surfactants and other related materials.

Other optional ingredients may be additionally used depending on the particular application. For example, well known auxiliaries and additives typically utilized in the coatings industry including, for example, foam inhibitors, levelling aids, pigments, dispersants such as pigment dispersing aids, dyes, UV absorbers, heat stabilizers, other stabilizing additives such as antioxidants. Other optional ingredients have been exemplified in the many previously incorporated references, and reference may be had thereto for further details.

### Preparation and Uses of the Curable Compositions

The curable compositions of the present invention are suitable for numerous uses including, for example, as coatings and adhesives, in decorative laminated boards, in the formation of crosslinked molded articles such as engineering composites, for textile and paper treatment, and in any other field in which traditional amine-formaldehyde resins are suitable for use.

The curable compositions may be prepared by admixing the various components via methods and in relative amounts which are recognizable by those of ordinary skill in the art in the relevant field depending upon the particular end use chosen. As a general rule, the resin component and the crosslinker component should preferably be admixed in an equivalents ratio (equivalents of reactive functionality) of from about 0.5:1 to about 2:1, and more preferably from about 0.8:1 to about 1.2:1.

An especially preferred use of the curable compositions in accordance with the present invention is in the coatings field. Any conventional type of coating may be prepared using the curable compositions described herein, including organic solvent based liquid coatings, waterbome coatings, powder coatings and high temperature coil coatings. In coatings applications, the weight amounts of the various reactive components will be dependent upon factors including, for example, the particular materials chosen, the presence of other reactive species as well as the desired end use. Based upon these variables and others, those of ordinary skill in the art should be able to adjust the composition of the coatings (including the relative amounts of the components) to achieve the desired effect.

Organic solvent based liquid coatings in accordance with the present invention may be prepared via conventional means by adding into a commonly used organic coatings solvent the components of the curable composition and the optional ingredients, if present, in any convenient order. In organic solvent based coatings, the systems are formulated to produce a solids content level suitable for convenient application with minimal material loss, preferably at a solids content level in the range of from about 20 weight percent to about 85 weight percent, and more preferably at a solids content level in the range of from about 45 weight percent to about 80 weight percent, depending on the method of application chosen.

Waterborne coating compositions in accordance with the present invention may be prepared by combining the components of the coating in any particular order, but it is preferred to do so by preparing a dispersible composition by substantially homogeneously mixing the coating components with a surface active material (which may be an inherent property of the resin component), then dispersing the dispersible composition in an aqueous medium, which may comprise solely water or may contain other components such as minor amounts of water-miscible co-solvents to ease dispersion or adjust viscosity. The waterborne coating compositions may be formulated to various solids contents, generally ranging from about 20% to about 75% by weight solids, but preferably in the range of from about 30% to about 55% by weight solids, depending on the method of application chosen.

Powder coating compositions in accordance with the present invention may be prepared by any well-known method, for example, by dry mixing the components in a mixer or blender followed by compounding in an extruder and granulating, grinding and then screening to obtain a powder of suitable mesh size for powder coating. For powder coatings applications, compositions containing solid crosslinker and backbone resin components are preferred. Alternatively, some or all of the components may be dissolved in a solvent such as methylene chloride and spray dried by well known techniques. Moreover, it may be preferable to masterbatch the crosslinking agent and the hydroxyl functional resin prior to mixing the optional components of the composition in a manner well known to a person skilled in the art.

The present coating compositions are utilized by applying the coating to a substrate then curing the so-applied coating to form crosslinked films. Liquid coatings may be applied, for example, by dipping, spraying, padding, brushing, flowcoating, electrocoating or electrostatic spraying. After application, the liquid carrier (e.g., organic solvent and/or water) is generally allowed to partially evaporate to produce a uniform coating on the substrate. Powder coatings may be applied, for example, by means such as a powder gun, electrostatic deposition or deposition from a fluidized bed. After deposition, the powder is typically heated usually to a temperature sufficient to cause the particles to soften, melt, flow and begin to cure.

Full curing of the present coating compositions (and curable compositions) requires elevated temperatures generally in the range of from about 25°C to about 450°C depending on the components as well as the end use application. In liquid coatings applications, the cure temperature is typically in the range of from about 80°C to about 160°C. In powder coatings applications, the cure temperature is typically in the range of from about 110°C to about 230°C, preferably from about 150°C to about 220°C, and most preferably from about 170°C to about 200°C. In coil coatings applications, the cure temperature is typically in the range of from about 250°C to about 450°C. Cure time preferably is in the range of from about 1 second to about 30 minutes but may vary depending on the temperature chosen for cure. For example, a fully cured coil coating may be obtained by either curing at 260°C for 1 minute or by curing at 417°C for 20 seconds. Typical cure times for liquid and powder coatings are in the in the range of from about 5 minutes to about 30 minutes.

The coating compositions of this invention may be formulated for use in numerous areas such as original equipment manufacturing (OEM) including automotive coatings, general industrial coatings including industrial maintenance coatings, architectural coatings and can coatings. They are usable as coatings for wire, appliances, automotive parts, furniture, pipes and machinery. The present systems can be used as 1 K coatings in applications such as automotive finishes, powder coatings, coil coatings including base coats and top coats. Suitable surfaces include metals such as steel and aluminum, plastics, wood, and glass.

The examples which follow are intended to be illustrative of certain preferred embodiments of the invention and are not to be construed to limit the invention in any manner. NMR spectra were obtained on a Varian Unity 300 Plus. IR spectra were obtained on a Digilab FTS 60A. LC/MS (Thermospray) was conducted on a Finnigen Mat TSQ-700 spectrometer. Melting points were measured on a Electrothermal Melting Point Apparatus. 2,2-Dimethoxy ethanal (DME) was obtained from Societe Francaise Hoechst as a 43 % solution in methyl tertiary butyl ether (MTBE) or as a 60 % solution in water. Melamine was obtained from Cytec Industries, West Paterson, N.J. Sodium bicarbonate and n-butanol (HPLC grade, 99.8 %) were obtained from Aldrich Chemical Company. Xylenes (ACS reagent), p-toluenesulfonic acid (practical), methanol (100 %, ACS reagent) and methylene chloride (ACS reagent) were obtained from J. T. Baker Chem. Co, Phillipsburg, N.J. Buffer solutions (Baxter calibrating buffer, pH 4, 7 and 10) were obtained from Baxter Diagnostics, Inc., Deerfield, IL. Water used was deionized water. All amounts are expressed as parts by weight, unless otherwise stated.

In some of the below examples, the DME utilized was purified by distilling a 43 % solution of DME in methyl tertiary butyl ether (MTBE) (552 g total) under reduced pressure (72 mm/Hg). The fraction boiling at 50-60°C was collected as a colorless liquid (174 g, yield 73%).

### EXAMPLE 1

DME (210 g total, 60 % in water, 1.2 moles DME) was placed in a one liter reaction flask and the pH was adjusted to 8.5 (buffer pH 10) with 20 % NaOH solution. Melamine (25.2 g, 0.2 moles) was then added and the mixture was stirred (mechanical stirring) at 50-60°C until it turned into a clear solution (about 30 min). The reaction was continued for an additional 10 min at 50-60°C. The resulting mixture was distilled under vacuum (6 mm/Hg, 50°C) for 1 hr to give a viscous liquid. After cooling the liquid to about 30°C, methanol (128 g, 4.0 moles) was added. The solution was then adjusted to pH 3.5 (buffer pH 4) with concentrated nitric acid and allowed to react at 40°C for 1 hr with vigorous stirring. The reaction was then stopped by adjusting the pH to 8.2 (buffer pH 7) with 50% NaOH solution. The solvent was removed under reduced pressure using a rotary evaporator (6 mm/Hg, 50°C, about 1 hr). The residue was dissolved in methanol (128 g, 4.0 moles) and the pH was adjusted to 4.5 (buffer pH 4) with concentrated nitric acid. The resulting solution was stirred at 60-65°C for 1 hr and the reaction was stopped by adjusting the pH to 7.2 (buffer pH 7) with 50% NaOH solution. The solvent was then removed under reduced pressure using a rotary evaporator (6 mm/Hg, 50°C). Water (200 ml) was then added to dissolve the residue and the solution was extracted with methylene chloride (3 x 150 ml). The organic layers were combined, washed with water (3 x 100 ml), and dried with anhydrous MgSO₄. Removal of the solvent under reduced pressure followed by crystallization in methanol gave substantially monomeric N,N',N"-tris-(1,2,2-trimethoxyeth-1-yl)melamine as white crystals (36 g, 38 % yield), m.p. 111-114°C (uncorrected). LC/MS (Thermospray, MH⁺): m/z calculated for C₁₈H₃₆O₉N₆+H 481. found 481. IR (KBr): 3332, 2943, 2833, 1583, 1565, 1450, 1373, 1189, 1078, 969, 944, 814 cm⁻¹. ¹H NMR (300 MHz, DMSO-d₆): δ 7.20 (m, NH), 5.25 (m, CH), 4.37 (m, CH), 3.30 (m, OCH3). ¹³C NMR (75 MHz, D₂O): δ 166.5, 103.4, 81.0, 55.6, 55.3.

The spectral data were consistent with the composition represented by the formula (Melamine)(DME)₃(Me)₃.

### EXAMPLE 2

Melamine (16.5 g, 0.13 moles), DME (freshly purified, 55 g, 0.53 moles), methanol (62 g, 1.95 moles) and p-toluenesulfonic acid (0.33 g, 0.002 moles) were placed in a 500 ml reaction flask under a nitrogen atmosphere. The mixture was stirred at reflux for 16 hr. Solvent was removed under reduced pressure using a rotary evaporator (6 mm/Hg, 40°C). The residue was dissolved in methylene chloride (200 ml) and washed with water (2 x 80 ml), sodium bicarbonate (2%, 80 ml) and water (2 x 80 ml). The organic layer was dried with anhydrous MgSO₄ and evaporated to dryness (6 mm/Hg, 40°C). Addition of xylenes gave a colorless solution of substantially monomeric N,N',N"-tris-(1,2,2-trimethoxyeth-1-yl)melamine (84 g) having a solids level of 51 % as determined by the pan solids method (heating at 105°C for 2 hr).

As in Example 1, the ¹³C NMR was consistent with the composition represented by the formula (Melamine)(DME)₃(Me)₃.

### EXAMPLE 3

Melamine (50 g, 0.4 moles), methanol (192 g, 6.0 moles), DME (freshly purified, 125 g, 1.2 moles) and p-toluenesulfonic acid (1 g, 0.006 moles) were placed in a 500 ml reaction flask under a nitrogen atmosphere and stirred at reflux for 24 hr. The reaction mixture was then cooled to room temperature and neutralized to pH 7 by 50% NaOH (approximately 1.5 ml). The solvent was removed under reduced pressure (50°C/6 mm/Hg) and the residue was dissolved in 1,2-propylene glycol monomethyl ether (PGME). The solution was filtered to give a colorless solution. ¹³C NMR of the product indicated an average composition of (Melamine)(DME)_{2.4}(Me)_{2.0}. A broad signal at 60 ppm, characteristic of an NH-CH-NH linkage, indicated that the product contained oligomeric forms at about 50 weight % level.

### EXAMPLE 4

Melamine (20 g, 0.16 moles), methanol (77 g, 2.4 moles), DME (freshly purified, 50 g, 0.48 moles) and concentrated nitric acid (2 g, 0.032 moles) were placed in a 500 ml reaction flask under a nitrogen atmosphere and stirred at reflux for 24 hr. The reaction mixture was then cooled to room temperature and neutralized to pH 7 (buffer pH 7) by 50 % NaOH. The solution was filtered to give a colorless solution. ¹³C NMR of the product indicated an average composition of (Melamine)(DME)_{2.3}(Me)_{1.6}. The broad signal at 60 ppm, characteristic of an NH-CH-NH linkage, indicated that the product contained oligomeric forms at about 66 weight % level.

### EXAMPLE 5

DME (113 g, 60% in water, 0.65 moles) and melamine (16.5 g, 0.13 moles) were placed in a 500 ml reaction flask and stirred at 70-80°C until the mixture became dear (about 30 min). The water was removed under reduced pressure using a rotary evaporator (6 mm/Hg, 40°C, about 30 min). Methanol (83 g, 2.6 moles) and p-toluenesufonic acid (0.34 g, 0.002 moles) were then added and the mixture was allowed to react at 70°C for 16 hr to give a yellow solution. The methanol was removed under vacuum (6 mm/Hg,40°C), methylene chloride (200 ml) was added, the solution was washed with water (4 x 60 ml) and thereafter dried with anhydrous MgSO₄. Removal of the methylene chloride under reduced pressure a viscous yellow liquid. ¹³C NMR spectrum indicated that the product was substantially monomeric, having a composition consistent with the formula (Melamine)(DME)₃(Me)₃.

### EXAMPLE 6

Melamine (45.4 g, 0.36 moles), n-butanol (400 g, 5.41 moles), DME (freshly purified, 150 g, 1.44 moles) and p-toluenesulfonic acid (0.93 g, 0.005 moles) were placed in an one litter reaction flask under a nitrogen atmosphere and stirred at 70-80°C for 24 hr. The solvent was removed under reduced pressure using a rotary evaporator (6 mm/Hg, 50°C). The residue was dissolved in methylene chloride (400 ml) and washed with water (2 x 300 ml), NaHCO₃ (2 x 200 ml) and water (2 x 300 ml). The organic layer was dried with anhydrous MgSO₄ and the solvent was removed under reduced pressure using a rotary evaporator. The residue was dissolved in 1,2-propylene glycol monomethyl ether (PGME) to give a viscous solution (204 g, 65 % solids by pan solids method). ¹³C NMR (300 MHz, in PGME) showed signals at 166.3 ppm for triazine carbons, 103.9 ppm for CH(OCH₃)₂, 80.5 ppm for CH(OBu)NH, 67.5 ppm for OCH₂, 54.4 ppm for OCH₃, 31.9 ppm for CH₂, 19.3 ppm for CH₂ and 14.1 ppm for CH₃, indicating an average composition of (Melamine)(DME)_{2.6}(Bu)_{2.6}.

### COMPARATIVE EXAMPLE

In a 500 ml reaction flask, DME (freshly purified, 79 g, 0.76 moles) was dissolved in xylenes (80 ml) under a nitrogen atmosphere. NaHCO₃ (2.4 g) and melamine (24 g, 0.19 moies) were then added and the resulting mixture was stirred at 50-55°C for 16 hr using a mechanical stirrer. Upon cooling to room temperature, the mixture had separated into two distinct layers. The xylenes layer was discarded. The residue was further extracted with xylenes (2 x 80 ml) and the xylenes layer was discarded. The residue was then dissolved in methylene chloride (100 ml) and filtered. The methylene chloride was then removed under reduced pressure using a rotary evaporator to give a colorless, viscous liquid (98 g). The crude product was soluble in ethyl acetate, methylene chloride and methyl ethyl ketone. IR (KBr) showed the formation of a trisubstituted triazine, H-bonded OH groups and secondary amines. As expected, the compound was unstable under the LC/MS (thermospray) analysis conditions and hence, a molecular ion peak was not observed. ¹³C NMR (300 MHz, CDCl₃) exhibited signals at 165.2 ppm for triazine carbons, 104.2 ppm for CH(OCH₃)₂, 74.2 ppm for NHCH(OH) and 55.6 ppm for OCH₃, indicating that the product was substantially monomeric, having a composition consistent with the formula (Melamine)(DME)₃.

### EXAMPLE 7

Coatings A-J were prepared by admixing the components with enough additional solvent to adjust the solids level to the percentage, as listed in Tables I-X below. Films derived from Coatings A-J were compared to films derived from comparative coatings (Comparative Coatings A-J) using a conventional-type methylated melamine-formaldehyde resin as crosslinker. The physical and resistance properties of the coatings and the comparatives are also provided in Tables I-X. These results show that curable systems based on the present compounds and compositions can be formulated to produce results comparable to those obtained from more conventional systems crosslinked with traditional amino-formaldehyde crosslinkers.

**TABLE I**

| | COATING A | COMPARATIVE A |
|---|---|---|
| Polyfunctional Material | Acryloid®AT400⁽¹⁾ | Acryloid®AT400 |
| Crosslinker | EXAMPLE 2 | Cymel®327 Resin⁽²⁾ |
| Polyfunctional Material/Crosslinker | 70/30 | 70/30 |
| Solids (on TRS)⁽³⁾ | 65 Weight % | 65 Weight % |
| Wire Cater Applicator | # 34 | # 34 |
| Flash Time | 15 minutes | 15 minutes |
| Cure Time | 30 minutes | 30 minutes |
| Solvent | Xylene | Xylene |
| Butanol | 10 Wt % on TRS | 10 Wt % on TRS |
| Substrate | B1000 CRS⁽⁴⁾ | B1000 CRS |
| Catalyst | None | None |
| | | |

| 150°C Cure | | |
|---|---|---|
| Mils (µm) | 0.91 (23.1) | 0.85 (21.6) |
| KHN₂₅ | 13.7 | 14.0 |
| MEK | 200+/200+ | 200+/200+ |
| Appearance | Good | Good |

| | | |
|---|---|---|
| ⁽¹⁾ a hydroxy functional acrylic resin of Rohm & Haas Co., Philadelphia, PA | | |
| ⁽²⁾ a high imino methylated melamine-formaldehyde resin of Cytec Industries, West Paterson, NJ | | |
| ⁽³⁾ Total Resin Solids | | |
| ⁽⁴⁾ Bonderite cold rolled steel panels | | |

**TABLE II**

| | COATING B | COMPARATIVE B |
|---|---|---|
| Polyfunctional Material | Acryloid®AT400 | Acryloid®AT400 |
| Crosslinker | EXAMPLE 2 | Cymel®327 Resin |
| Polyfunctional Material/Crosslinker | 70/30 | 70/30 |
| Solids (on TRS) | 65 Weight % | 65 Weight % |
| Wire Cater Applicator | # 34 | # 34 |
| Flash Time | 15 minutes | 15 minutes |
| Cure Time | 30 minutes | 30 minutes |
| Solvent | Xylene | Xylene |
| Butanol | 10 Wt % on TRS | 10 Wt % on TRS |
| Substrate | B1000 CRS | B1000 CRS |
| Catalyst (PTSA)⁽⁵⁾ | 0.8 Wt % | 0.8 Wt % |
| | | |

| 100°C Cure | | |
|---|---|---|
| Mils (µm) | 0.91 (23.1) | 0.85 (21.6) |
| KHN₂₅ | 8.7 | 12.0 |
| MEK | 5/63 | 200+/200+ |
| Appearance | Good | Good |
| | | |

| 125°C Cure | | |
|---|---|---|
| Mils (µm) | 0.93 (23.6) | 0.88 (22.4) |
| KHN₂₅ | 13.0 | 15.3 |
| MEK | 200+/200+ | 200+/200+ |
| Appearance | Good | Good |
| | | |

| 150°C Cure | | |
|---|---|---|
| Mils (µm) | 0.81 (20.6) | 0.87 (22.1) |
| KHN₂₅ | 13.7 | 15.3 |
| MEK | 200+/200+ | 200+/200+ |
| Appearance | Good | Good |

| | | |
|---|---|---|
| ⁽⁵⁾ p-Toluenesulfonic Acid, Weight % based on Total Resin Solids (TRS) | | |

**TABLE III**

| | COATING C | COMPARATIVE C |
|---|---|---|
| Polyfunctional Material | Acryloid®AT400 | Acryloid®AT400 |
| Crosslinker | EXAMPLE 3 | Cymel®327 Resin |
| Polyfunctional Material/Crosslinker | 70/30 | 70/30 |
| Solids (on TRS) | 62.3 Weight % | 62.3 Weight % |
| Wire Cater Applicator | # 40 | # 40 |
| Flash Time | 15 minutes | 15 minutes |
| Cure Time | 30 minutes | 30 minutes |
| Solvent | Xylene | Xylene |
| Butanol | 10 Wt % on TRS | 10 Wt % on TRS |
| Substrate | B1000 CRS | B1000 CRS |
| Catalyst | None | None |
| | | |

| 125°C Cure | | |
|---|---|---|
| Mils (µm) | 1.08 (27.4) | 1.09 (27.7) |
| KHN₂₅ | 5.7 | 12.1 |
| MEK | 25/200 | 100/200+ |
| | | |

| 150°C Cure | | |
|---|---|---|
| Mils (µm) | 1.18 (30.0) | 1.03 (26.2) |
| KHN₂₅ | 11.1 | 13.2 |
| MEK | 200+/200+ | 200+/200+ |

**TABLE IV**

| | COATING D | COMPARATIVE D |
|---|---|---|
| Polyfunctional Material | Acryloid®AT400 | Acryloid®AT400 |
| Crosslinker | EXAMPLE 3 | Cymel®327 Resin |
| Polyfunctional Material/Crosslinker | 70/30 | 70/30 |
| Solids (on TRS) | 62.3 Weight % | 62.3 Weight % |
| Wire Cater Applicator | # 40 | # 40 |
| Flash Time | 15 minutes | 15 minutes |
| Cure Time | 30 minutes | 30 minutes |
| Solvent | Xylene | Xylene |
| Butanol | 10 Wt % on TRS | 10 Wt % on TRS |
| Substrate | B1000 CRS | B1000 CRS |
| Catalyst (PTSA) | 0.2 Wt % | 0.2 Wt % |
| | | |

| 125°C Cure | | |
|---|---|---|
| Mils (µm) | 1.11 (28.2) | 1.09 (27.7) |
| KHN₂₅ | 9.8 | 12.4 |
| MEK | 25/200+ | 200+/200+ |
| | | |

| 150°C Cure | | |
|---|---|---|
| Mils (µm) | 1.16(29.5) | 0.98 (24.9) |
| KHN₂₅ | 10.6 | 15.3 |
| MEK | 200+/200+ | 200+/200+ |

**TABLE V**

| | COATING E | COMPARATIVE E |
|---|---|---|
| Polyfunctional Material | Acryloid®AT400 | Acryloid®AT400 |
| Crosslinker | EXAMPLE 3 | Cymel®327 Resin |
| Polyfunctional Material/Crosslinker | 70/30 | 70/30 |
| Solids (on TRS) | 62.3 Weight % | 62.3 Weight % |
| Wire Cater Applicator | # 40 | # 40 |
| Flash Time | 15 minutes | 15 minutes |
| Cure Time | 30 minutes | 30 minutes |
| Solvent | Xylene | Xylene |
| Butanol | 10 Wt % on TRS | 10 Wt % on TRS |
| Substrate | B1000 CRS | B1000 CRS |
| Catalyst (PTSA) | 0.4 Wt % | 0.4 Wt % |
| | | |

| 125°C Cure | | |
|---|---|---|
| Mils (µm) | 1.18 (30.0) | 1.18 (30.0) |
| KHN₂₅ | 10.1 | 13.7 |
| MEK | 25/200+ | 25/200+ |
| | | |

| 150°C Cure | | |
|---|---|---|
| Mils (µm) | 1.11 (28.2) | 1.21 (30.7) |
| KHN₂₅ | 10.8 | 14.2 |
| MEK | 200+/200+ | 200+/200+ |

**TABLE VI**

| | COATING F | COMPARATIVE F |
|---|---|---|
| Polyfunctional Material | Acryloid®AT400 | Acryloid®AT400 |
| Crosslinker | EXAMPLE 3 | Cymel®327 Resin |
| Polyfunctional Material/Crosslinker | 70/30 | 70/30 |
| Solids (on TRS) | 62.3 Weight % | 62.3 Weight % |
| Wire Cater Applicator | # 40 | # 40 |
| Flash Time | 15 minutes | 15 minutes |
| Cure Time | 30 minutes | 30 minutes |
| Solvent | Xylene | Xylene |
| Butanol | 10 Wt % on TRS | 10 Wt % on TRS |
| Substrate | B1000 CRS | B1000 CRS |
| Catalyst (Cycat®296-9)⁽⁶⁾ | 1.0 Wt % | 1.0 Wt % |
| | | |

| 125°C Cure | | |
|---|---|---|
| Mils (µm) | 1.12 (28.4) | 1.15 (29.2) |
| KHN₂₅ | 8.4 | 13.7 |
| MEK | 25/60 | 200+/200+ |
| | | |

| 150°C Cure | | |
|---|---|---|
| Mils (µm) | 1.1 (27.9) | 1.2 (30.5) |
| KHN₂₅ | 11.7 | 14.0 |
| MEK | 200+/200+ | 200+/200+ |

| | | |
|---|---|---|
| ⁽⁶⁾ a phosphoric acid derivative catalyst of Cytec Industries, West Paterson, NJ | | |

**TABLE VII**

| | COATING G | COMPARATIVE G |
|---|---|---|
| Polyfunctional Material | Acryloid®AT400 | Acryloid®AT400 |
| Crosslinker | EXAMPLE 6 | Cymel®327 Resin |
| Polyfunctional Material/Crosslinker | 70/30 | 70/30 |
| Solids (on TRS) | 62.3 Weight % | 62.3 Weight % |
| Wire Cater Applicator | # 40 | # 40 |
| Flash Time | 15 minutes | 15 minutes |
| Cure Time | 30 minutes | 30 minutes |
| Solvent | Xylene | Xylene |
| Butanol | 10 Wt % on TRS | 10 Wt % on TRS |
| Substrate | B1000 CRS | B1000 CRS |
| Catalyst | None | None |
| | | |

| 125°C Cure | | |
|---|---|---|
| Mils (µm) | 1.15 (29.2) | 1.09 (27.7) |
| KHN₂₅ | 1.1 | 12.1 |
| MEK | 1/22 | 100/200+ |
| | | |

| 150°C Cure | | |
|---|---|---|
| Mils (µm) | 1.18 (30.0) | 1.03 (26.2) |
| KHN₂₅ | 9.8 | 13.2 |
| MEK | 200+/200+ | 200+/200+ |

**TABLE VIII**

| | COATING H | COMPARATIVE H |
|---|---|---|
| Polyfunctional Material | Acryloid®AT400 | Acryloid®AT400 |
| Crosslinker | EXAMPLE 6 | Cymel®327 Resin |
| Polyfunctional Material/Crosslinker | 70/30 | 70/30 |
| Solids (on TRS) | 62.3 Weight % | 62.3 Weight % |
| Wire Cater Applicator | # 40 | # 40 |
| Flash Time | 15 minutes | 15 minutes |
| Cure Time | 30 minutes | 30 minutes |
| Solvent | Xylene | Xylene |
| Butanol | 10 Wt % on TRS | 10 Wt % on TRS |
| Substrate | B1000 CRS | B1000 CRS |
| Catalyst (PTSA) | 0.2 Wt % | 0.2 Wt % |
| | | |

| 125°C Cure | | |
|---|---|---|
| Mils (µm) | 1.13 (28.7) | 1.09 (27.7) |
| KHN₂₅ | 8.2 | 12.4 |
| MEK | 25/150 | 200+/200+ |
| | | |

| 150°C Cure | | |
|---|---|---|
| Mils (µm) | 1.09 (27.7) | 0.98 (24.9) |
| KHN₂₅ | 10.5 | 15.3 |
| MEK | 200+/200+ | 200+/200+ |

**TABLE IX**

| | COATING I | COMPARATIVE I |
|---|---|---|
| Polyfunctional Material | Acryloid®AT400 | Acryloid®AT400 |
| Crosslinker | EXAMPLE 6 | Cymel®327 Resin |
| Polyfunctional Material/Crosslinker | 70/30 | 70/30 |
| Solids (on TRS) | 62.3 Weight % | 62.3 Weight % |
| Wire Cater Applicator | # 40 | # 40 |
| Flash Time | 15 minutes | 15 minutes |
| Cure Time | 30 minutes | 30 minutes |
| Solvent | Xylene | Xylene |
| Butanol | 10 Wt % on TRS | 10 Wt % on TRS |
| Substrate | B1000 CRS | B1000 CRS |
| Catalyst (PTSA) | 0.4 Wt % | 0.4 Wt % |
| | | |

| 125°C Cure | | |
|---|---|---|
| Mils (µm) | 1.10 (27.9) | 1.18 (30.0) |
| KHN₂₅ | 10.0 | 13.7 |
| MEK | 25/200+ | 100/200+ |
| | | |

| 150°C Cure | | |
|---|---|---|
| Mils (µm) | 0.98 (24.9) | 1.21 (30.7) |
| KHN₂₅ | 10.3 | 14.2 |
| MEK | 200+/200+ | 200+/200+ |

**TABLE X**

| | COATING J | COMPARATIVE J |
|---|---|---|
| Polyfunctional Material | Acryloid®AT400 | Acryloid®AT400 |
| Crosslinker | EXAMPLE 6 | Cymel®327 Resin |
| Polyfunctional Material/Crosslinker | 70/30 | 70/30 |
| Solids (on TRS) | 62.3 Weight % | 62.3 Weight % |
| Wire Cater Applicator | # 40 | # 40 |
| Flash Time | 15 minutes | 15 minutes |
| Cure Time | 30 minutes | 30 minutes |
| Solvent | Xylene | Xylene |
| Butanol | 10 Wt % on TRS | 10 Wt % on TRS |
| Substrate | B1000 CRS | B1000 CRS |
| Catalyst (CYCAT®296-9) | 1.0 Wt % | 1.0 Wt % |
| | | |

| 125°C Cure | | |
|---|---|---|
| Mils (µm) | 1.05 (26.7) | 1.15 (29.2) |
| KHN₂₅ | 10.3 | 13.7 |
| MEK | 25/200+ | 200+/200+ |
| | | |

| 150°C Cure | | |
|---|---|---|
| Mils (µm) | 1.0 (25.4) | 1.2 (30.5) |
| KHN₂₅ | 10.3 | 14.0 |
| MEK | 200+/200+ | 200+/200+ |

### EXAMPLE 8

Coating K was prepared by admixing the components with enough additional solvent to adjust the solids level to the percentage, as listed in Table XI below. A film derived from Coating K was compared to a film derived from a comparative coating (Comparative Coatings K) using the crosslinker from the Comparative Example - which is a melamine/DME condensate similar to the compounds of the present invention but containing no activated ether groups (none of the 1-hydroxy groups have been alkylated). The physical and resistance properties of the coating and the comparative, provided below in Table XI, clearly show the necessity of these activated ether groups as required by the present invention.

**TABLE XI**

| | COATING K | COMPARATIVE K |
|---|---|---|
| Polyfunctional Material | Acryloid®AT400 | Acryloid®AT400 |
| Crosslinker | EXAMPLE 2 | COMPARATIVE EXAMPLE |
| Polyfunctional Material/Crosslinker | 70/30 | 70/30 |
| Solids (on TRS) | 65 Weight % | 65 Weight % |
| Wire Cater Applicator | # 34 | # 34 |
| Flash Time | 15 minutes | 15 minutes |
| Cure Time | 30 minutes | 30 minutes |
| Solvent | Xylene | Xylene |
| Butanol | 10 Wt % on TRS | 10 Wt % on TRS |
| Substrate | B1000 CRS | B1000 CRS |
| Catalyst (PTSA) | 0.8 Wt % | 0.8 Wt % |
| | | |

| 100°C Cure | | |
|---|---|---|
| Mils (µm) | 0.91 (23.1) | 0.83 (21.1) |
| KHN₂₅ | 8.7 | 1.8 |
| MEK | 5/63 | 1/5 |
| Appearance | Good | Poor |
| | | |

| 125°C Cure | | |
|---|---|---|
| Mils (µm) | 0.93 (23.6) | 0.80 (20.3) |
| KHN₂₅ | 13.0 | 1.8 |
| MEK | 200+/200+ | 1/5 |
| Appearance | Good | Poor |
| | | |

| 150°C Cure | | |
|---|---|---|
| Mils (µm) | 0.81 (20.6) | 0.75 (19.1) |
| KHN₂₅ | 13.7 | 1.3 |
| MEK | 200+/200+ | 1/ 5 |
| Appearance | Good | Poor |

### EXAMPLES 9 AND 10

Waterbome Coatings L and M were prepared by admixing the components as listed in Tables XII-XIII below. Films derived from Waterbome Coatings L and M were compared to films derived from comparative coatings (Comparative Coatings L and M) using a conventional-type methylated melamine-formaldehyde resin as crosslinker. The physical and resistance properties of the coatings and the comparatives are also provided in Tables XII-XIII. These results show that curable waterbome systems based on the present compounds and compositions can be formulated to produce results comparable to those obtained from more conventional systems crosslinked with traditional amino-formaldehyde crosslinkers.

**TABLE XII**

| WATERBORNE COATINGS | COATING L | COMPARATIVE L |
|---|---|---|
| Polyfunctional Material | Rhoplex®AC1024⁽⁷⁾ | Rhoplex®AC 1024 |
| Crosslinker | Example 2 | Cymel®327 Resin |
| Polyfunctional Material/Crosslinker | 50/50 | 50/50 |
| Solids (on TRS) | 50 Weight % | 50 Weight % |
| Wire Cater Applicator | # 34 | # 34 |
| Flash Time | 15 minutes | 15 minutes |
| Cure Time | 30 minutes | 30 minutes |
| Substrate | B1000 CRS | B1000 CRS |
| Catalyst (PTSA) | 0.8 Wt % | 0.8 Wt % |
| | | |

| 100°C Cure | | |
|---|---|---|
| Mils (µm) | 1.12(28.4) | 1.31 (33.3) |
| KHN₂₅ | 10.9 | 11.0 |
| MEK | 150/200+ | 150/200+ |
| | | |

| 125°C Cure | | |
|---|---|---|
| Mils (µm) | 1.07 (27.2) | 1.13 (28.7) |
| KHN₂₅ | 15.8 | 8.0 |
| MEK | 200+/200+ | 200+/200+ |
| | | |

| 150°C Cure | | |
|---|---|---|
| Mils (µm) | 1.03 (26.2) | 1.16 (29.5) |
| KHN₂₅ | 18.2 | 18.2 |
| MEK | 200+/200+ | 200+/200+ |

| | | |
|---|---|---|
| ⁽⁷⁾ a hydroxy functional acrylic emulsion resin of Rohm & Haas Company, Philadelphia, PA | | |

**TABLE XIII**

| YELLOWING ON OVERBAKE WATERBORNE COATINGS | COATING M | COMPARATIVE M |
|---|---|---|
| Polyfunctional Material | Rhoplex®AC1024 | Rhoplex®AC1024 |
| Crosslinker | Example 2 | Cymel®327 Resin |
| Polyfunctional Material/Crosslinker | 50/50 | 50/50 |
| Solids (on TRS) | 50 Weight % | 50 Weight % |
| Wire Cater Applicator | # 34 | # 34 |
| Flash Time | 15 minutes | 15 minutes |
| Cure Time | 30 minutes | 30 minutes |
| Substrate | B1000 CRS | B1000 CRS |
| Prime | White Basecoat | White Basecoat |
| Catalyst (PTSA) | 0.8 Wt % | 0.8 Wt % |
| | | |

| 150°C Cure | | |
|---|---|---|
| Mils (µm) | 1.03 (26.2) | 1.16 (29.5) |
| KHN₂₅ | 18.2 | 18.2 |
| MEK | 200+/200+ | 200+/200+ |
| Initial Yellow Index | -1.7 | -1.6 |
| Change in Yellow Index on Overbake: | | |
| 1.8 hrs at 150°C | 1.5 | 1.2 |
| 3.8 hrs at 150°C | 3.9 | 4.4 |
| 8.0 hrs at 150°C | 8.2 | 9.6 |

Although the present invention is described with reference to certain preferred embodiments, it is apparent that modifications and variations thereof may be made by those skilled in the art without departing from the scope of this invention as defined by the appended claims.

## Claims

1. An N-1,2,2-trihydrocarbyloxyethyl derivative of an amino compound, comprising the reaction product of:
(i) an amino compound having at least two =NH groups, selected from the group consisting of amino-1,3,5-triazines, glycolurils and oligomers thereof,
(ii) a 2,2-dihydrocarbyloxy ethanal and
(iii) a hydrocarbylol, excluding polyols having 2 or more hydroxyl groups
the reaction product containing, on average, at least 1.25 moles of combined 2,2-dihydrocarbyloxy ethanal per mole of amino compound, and at least about 2.0 1,2,2-trihydrocarbyloxyethyl groups per molecule of derivative.

2. The N-1,2,2-trihydrocarbyloxyethyl derivative of claim 1, characterized in that the amino compound is selected from the group consisting of amino-1,3,5-triazines and glycolurils of the general formulas (IV) and (V): 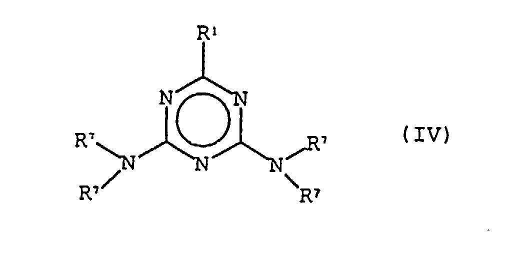 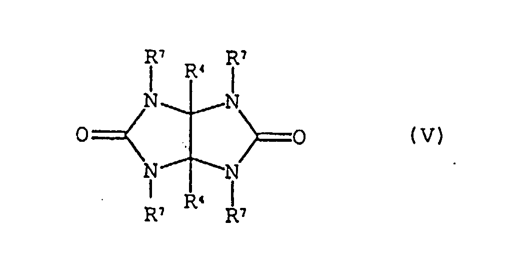 wherein
R¹ is selected from H, a hydrocarbyl and -N(R⁷)₂,
each R⁷ is independently selected from H and a hydrocarbyl, with the proviso that at least two groups R⁷ are H, and preferably that all R⁷ groups are H, and
each R⁴ is independently selected from H and a hydrocarbyl, and preferably that all R⁴ groups are H.

3. The N-1,2,2-trihydrocarbyloxyethyl derivative of claim 2, characterized in that the amino compound is of the general formula (IV) and all R⁷ groups are H.

4. The N-1,2,2-trihydrocarbyloxyethyl derivative of claim 3, characterized in that the derivative is a guanamine derivative containing on average from about 1.5 to about 2.0 moles of combined 2,2-dihydrocarbyloxy ethanal per mole of guanamine.

5. The N-1,2,2-trihydrocarbyloxyethyl derivative of claim 3, characterized in that the derivative is a melamine derivative containing on average from about 2.0 to about 3.0 moles of combined 2,2-dihydrocarbyloxy ethanal per mole of melamine.

6. The N-1,2,2-trihydrocarbyloxyethyl derivative of claim 2, characterized in that the amino compound is of the general formula (V), all R⁴ groups are H and all R⁷ groups are H.

7. The N-1,2,2-trihydrocarbyloxyethyl derivative of claim 6, characterized in that the derivative is a glycoluril derivative containing on average from about 2.0 to about 4.0 moles of combined 2,2-dihydrocarbyloxy ethanal per mole of glycoluril.

8. The N-1,2,2-trihydrocarbyloxyethyl derivative of claim 1, characterized in that the 2,2-dihydrocarbyloxy ethanal has the general formula (VI): 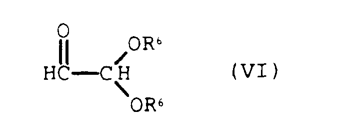 wherein
each R⁶ is independently a hydrocarbyl, or together form a hydrocarbylene bridge.

9. The N-1,2,2-trihydrocarbyioxyethyl derivative of claim 8, characterized in that each R⁶ is independently an alkyl of 1 to 8 carbon atoms or an alkenyl of 1 to 8 carbon atoms, or both R⁶ groups together form an alkylene bridge of 1 to 8 carbon atoms.

10. The N-1,2,2-trihydrocarbyloxyethyl derivative of claim 2, characterized in that the 2,2-dihydrocarbyloxy ethanal has the general formula (VI): 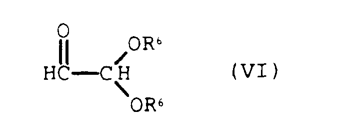 wherein
each R⁶ is independently a hydrocarbyl, or together form a hydrocarbylene bridge.

11. The N-1,2,2-trihydrocarbyloxyethyl derivative of claim 1, characterized in that the hydrocarbylol is a hydroxy group-containing compound having 1 to 20 carbon atoms.

12. The N-1,2,2-trihydrocarbyloxyethyl derivative of claim 2, characterized in that the hydrocarbylol is a hydroxy group-containing compound having 1 to 20 carbon atoms.

13. The N-1,2,2-trihydrocarbyloxyethyl derivative of claim 10, characterized in that the hydrocarbylol is a hydroxy group-containing compound having 1 to 20 carbon atoms.

14. A compound comprising an amino core having pendant therefrom at least two 1,2,2-trihydrocarbyloxyethyl groups, of the following general formula (I) or (II): 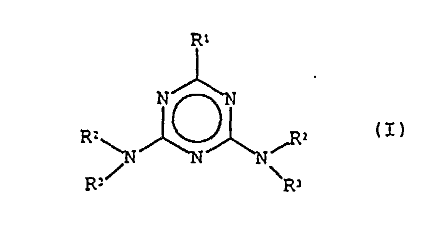 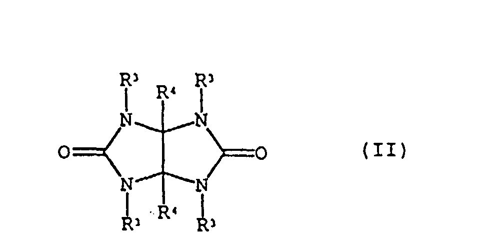 wherein
R¹ is selected from H, a hydrocarbyl and -N(R²)(R³);
each R² is independently selected from H and a hydrocarbyl;
each R³ is independently selected from H, a hydrocarbyl and an R group;
each R⁴ is independently selected from H and a hydrocarbyl; and
each R group is independently a group of the general formula (III) 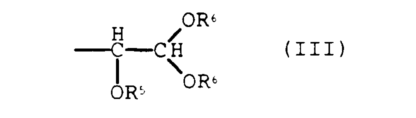
wherein
each R⁵ is independently selected from H and a hydrocarbyl, and each R⁶ is independently a hydrocarbyl, or together form a hydrocarbylene bridge;
with the proviso that, per molecule, at least two of the R³ groups are independently an R group, and at least two R⁵ groups are independently a hydrocarbyl.

15. The compound of claim 14, characterized in that the compound is of the general formula (I); each R² is H; each R³ is an R group; each R⁵ is independently selected from H and an alkyl of 1 to 8 carbon atoms, with the proviso that at least two R⁵ groups are an alkyl of 1 to 8 carbon atoms; and each R⁶ is independently an alkyl of 1 to 8 carbon atoms or an alkenyl of 1 to 8 carbon atoms, or both R⁶ groups on each group of the formula (III) together form an alkylene bridge of 1 to 8 carbon atoms.

16. The compound of claim 14, characterized in that the compound is of the general formula (II); each R⁴ is H; each R³ is selected from H and an R group, with the proviso that at least two of the R³ groups are an R group; each R⁵ is selected from H and an alkyl of 1 to 8 carbon atoms, with the proviso that at least two R⁵ groups are an alkyl of 1 to 8 carbon atoms; and each R⁶ is independently an alkyl of 1 to 8 carbon atoms or an alkenyl of 1 to 8 carbon atoms, or both R⁶ groups on each group of the formula (III) together form an alkylene bridge of 1 to 8 carbon atoms.

17. A process of preparing an N-1,2,2-trihydrocarbyloxyethyl derivative of an amino compound as set forth in any one of claims 1-13, characterized in that the process comprises the step of contacting:
(i) an amino compound having at least two =NH groups, selected from the group consisting of amino-1,3,5-triazines, glycolurils and oligomers thereof,
(ii) a 2,2-dihydrocarbyloxy ethanal and
(iii) a hydrocarbylol, excluding polyols having 2 or more hydroxyl groups
under conditions so as to result in a derivative containing, on average, at least 1.25 moles of combined 2,2-dihydrocarbyloxy ethanal per mole of amino compound, and at least about 2.0 1,2,2-trihydrocarbyloxyethyl groups per molecule of derivative.

18. The process of claim 17, characterized in that in a first step (i) and (ii) are contacted in the presence of a basic catalyst to produce a 1-hydroxy-2,2-dihydrocarbyloxyethyl derivative intermediate which, in a second step, is contact with (iii) under acidic conditions to produce the N-1,2,2-trihydrocarbyloxyethyl derivative of the amino compound.

19. The process of claim 17, characterized in that (i), (ii) and (iii) are concurrently contacted in the presence of an acid catalyst to directly produce the N-1,2,2-trihydrocarbyloxyethyl derivative of the amino compound.

20. A curable composition comprising:
(a) a crosslinker component comprising an N-1,2,2-trihydrocarbyloxyethyl derivative of an amino compound as set forth in any one of claims 1-16; and
(b) a resin component comprising a compound containing at least two groups capable of reacting with the 1,2,2-trihydrocarbyloxyethyl groups of (a).

21. A substrate coated with a crosslinked film derived from the curable composition according to claim 20.

## Patentansprüche

1. N-1,2,2-Trihydrocarbyloxyethylderivat einer Aminoverbindung, umfassend das Reaktionsprodukt aus:
(i) einer Aminoverbindung mit mindestens zwei =NH-Gruppen, ausgewählt aus der Gruppe, die aus Amino-1,3,5-Triazinen, Glykolurilen und Oligomeren davon besteht,
(ii) einem 2,2-Dihydrocarbyloxyethanal und
(iii) einem Hydrocarbylol, ausgenommen Polyole mit 2 oder mehr Hydroxylgruppen,
wobei das Reaktionsprodukt im Durchschnitt mindestens 1,25 Mol gebundenes 2,2-Dihydrocarbyloxyethanal pro Mol Aminoverbindung und mindestens etwa 2,0 1,2,2-Trihydrocarbyloxyethylgruppen pro Molekül des Derivats enthält.

2. N-1,2,2-Trihydrocarbyloxyethylderivat nach Anspruch 1, dadurch gekennzeichnet, daß die Aminoverbindung aus der Gruppe ausgewählt ist, die aus Amino-1,3,5-Triazinen und Glykolurilen der allgemeinen Formeln (IV) und (V) besteht: 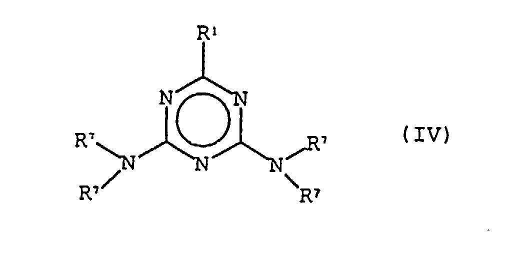 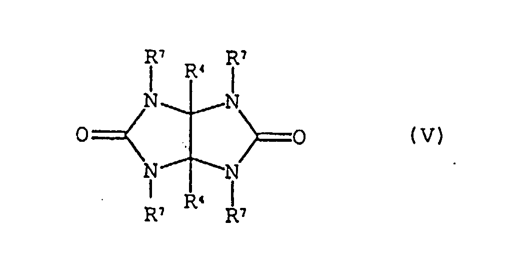 wobei
R¹ aus einem H, einem Hydrocarbyl und -N(R⁷)₂ ausgewählt ist,
jedes R⁷ unabhängig aus H und einem Hydrocarbyl ausgewählt ist, unter der Bedingung, daß mindestens zwei R⁷-Gruppen H sind und vorzugsweise, daß alle R⁷-Gruppen H sind, und
jedes R⁴ unabhängig aus H und einem Hydrocarbyl ausgewählt ist, und vorzugsweise, daß alle R⁴-Gruppen H sind.

3. N-1,2,2-Trihydrocarbyloxyethylderivat nach Anspruch 2, dadurch gekennzeichnet, daß die Aminoverbindung die allgemeine Formel (IV) aufweist und daß alle R⁷-Gruppen H sind.

4. N-1,2,2-Trihydrocarbyloxyethylderivat nach Anspruch 3, dadurch gekennzeichnet, daß das Derivat ein Guanaminderivat ist, das im Durchschnitt etwa 1,5 bis etwa 2,0 Mol gebundenes 2,2-Dihydrocarbyloxyethanal pro Mol Guanamin enthält.

5. N-1,2,2-Trihydrocarbyloxyethylderivat nach Anspruch 3, dadurch gekennzeichnet, daß das Derivat ein Melaminderivat ist, das im Durchschnitt etwa 2,0 bis etwa 3,0 Mol gebundenes 2,2-Dihydrocarbyloxyethanal pro Mol Melamin enthält.

6. N-1,2,2-Trihydrocarbyloxyethylderivat nach Anspruch 2, dadurch gekennzeichnet, daß die Aminoverbindung die allgemeine Formel (V) aufweist, sämtliche R⁴-Gruppen H sind und sämtliche R⁷-Gruppen H sind.

7. N-l,2,2-Trihydrocarbyloxyethylderivat nach Anspruch 6, dadurch gekennzeichnet, daß das Derivat ein Glykolurilderivat ist, das im Durchschnitt etwa 2,0 bis etwa 4,0 Mol gebundenes 2,2-Dihydrocarbyloxyethanal pro Mol Glykoluril enthält.

8. N-1,2,2-Trihydrocarbyloxyethylderivat nach Anspruch 1, dadurch gekennzeichnet, daß das 2,2-Dihydrocarbyloxyethanal die allgemeine Formel (VI) aufweist: 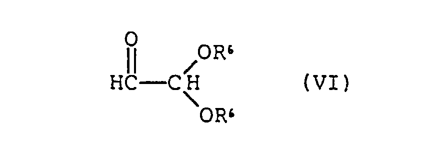 wobei
wobei wobei jedes R⁶ unabhängig ein Hydrocarbyl ist oder sie zusammen eine Hydrocarbylenbrücke bilden.

9. N-1,2,2-Trihydrocarbyloxyethylderivat nach Anspruch 8, dadurch gekennzeichnet, daß jedes R⁶ unabhängig ein Alkyl mit 1 bis 8 Kohlenstoffatomen oder ein Alkenyl mit 1 bis 8 Kohlenstoffatomen ist oder daß beide R⁶-Gruppen zusammen eine Alkylenbrücke mit 1 bis 8 Kohlenstoffatomen bilden.

10. N-1,2,2-Trihydrocarbyloxyethylderivat nach Anspruch 2, dadurch gekennzeichnet, daß das 2,2-Dihydrocarbyloxyethanal die allgemeine Formel (VI) aufweist: 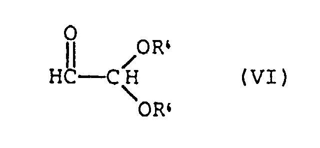 wobei
jedes R⁶ unabhängig ein Hydrocarbyl ist oder sie zusammen eine Hydrocarbylenbrücke bilden.

11. N-1,2,2-Trihydrocarbyloxyethylderivat nach Anspruch 1, dadurch gekennzeichnet, daß das Hydrocarbylol eine Verbindung mit 1 bis 20 Kohlenstoffatomen ist, die eine Hydroxygruppe enthält.

12. N-1,2,2-Trihydrocarbyloxyethylderivat nach Anspruch 2, dadurch gekennzeichnet, daß das Hydrocarbylol eine Verbindung mit 1 bis 20 Kohlenstoffatomen ist, die eine Hydroxygruppe enthält.

13. N-1,2,2-Trihydrocarbyloxyethylderivat nach Anspruch 10, dadurch gekennzeichnet, daß das Hydrocarbylol eine Verbindung mit 1 bis 20 Kohlenstoffatomen ist, die eine Hydroxygruppe enthält.

14. Verbindung, die einen Aminokern umfaßt, der als Seitengruppe davon mindestens zwei 1,2,2-Trihydrocarbyloxyethylgruppen der folgenden allgemeinen Formel (I) oder (II) aufweist: 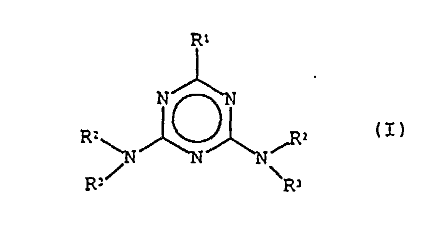 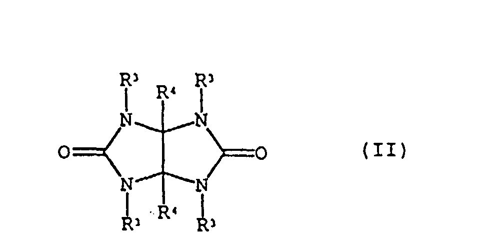 wobei
R¹ aus einem H, einem Hydrocarbyl und -N(R²)(R³) ausgewählt ist;
jedes R² unabhängig aus H und einem Hydrocarbyl ausgewählt ist;
jedes R³ unabhängig aus H, einem Hydrocarbyl und einer R-Gruppe ausgewählt ist;
jedes R⁴ unabhängig aus H und einem Hydrocarbyl ausgewählt ist; und
jede R-Gruppe unabhängig eine Gruppe der allgemeinen Formel (III) ist 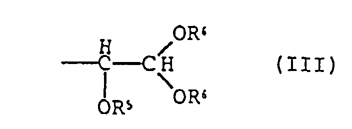
wobei
jedes R⁵ unabhängig aus H und einem Hydrocarbyl ausgewählt ist und jedes R⁶ unabhängig ein Hydrocarbyl ist oder sie zusammen eine Hydrocarbylenbrücke bilden;
unter der Bedingung, daß pro Molekül mindestens zwei der R³-Gruppen unabhängig eine R-Gruppe sind und mindestens zwei R⁵-Gruppen unabhängig ein Hydrocarbyl sind.

15. Verbindung nach Anspruch 14, dadurch gekennzeichnet, daß die Verbindung die allgemeine Formel (I) aufweist; jedes R² H ist; jedes R³ eine R-Gruppe ist; jedes R⁵ unabhängig aus H und einem Alkyl mit 1 bis 8 Kohlenstoffatomen ausgewählt ist, unter der Bedingung, daß mindestens zwei R⁵-Gruppen ein Alkyl mit 1 bis 8 Kohlenstoffatomen sind; und daß jedes R⁶ unabhängig ein Alkyl mit 1 bis 8 Kohlenstoffatomen oder ein Alkenyl mit 1 bis 8 Kohlenstoffatomen ist oder daß beide R⁶-Gruppen in jeder Gruppe der Formel (III) zusammen eine Alkylenbrücke mit 1 bis 8 Kohlenstoffatomen bilden.

16. Verbindung nach Anspruch 14, dadurch gekennzeichnet, daß die Verbindung die allgemeine Formel (II) aufweist; jedes R⁴ H ist; jedes R³ aus H und einer R-Gruppe ausgewählt ist unter der Bedingung, daß mindestens zwei der R³-Gruppen eine R-Gruppe sind; jedes R⁵ aus H und einem Alkyl mit 1 bis 8 Kohlenstoffatomen ausgewählt ist unter der Bedingung, daß mindestens zwei R⁵-Gruppen ein Alkyl mit 1 bis 8 Kohlenstoffatomen sind; und jedes R⁶ unabhängig ein Alkyl mit 1 bis 8 Kohlenstoffatomen oder ein Alkenyl mit 1 bis 8 Kohlenstoffatomen ist oder beide R⁶-Gruppen in jeder Gruppe der Formel (III) zusammen eine Alkylenbrücke mit 1 bis 8 Kohlenstoffatomen bilden.

17. Verfahren zur Herstellung eines N-1,2,2-Trihydrocarbyloxyethylderivats einer Aminoverbindung nach einem der Ansprüche 1 - 13, dadurch gekennzeichnet, daß das Verfahren den Schritt umfaßt, miteinander in Kontakt zu bringen:
(i) eine Aminoverbindung, die mindestens zwei =NH-Gruppen aufweist, ausgewählt aus der Gruppe, die aus Amino-1,3,5-Triazinen, Glykolurilen und Oligomeren davon besteht,
(ii) ein 2,2-Dihydrocarbyloxyethanal und
(iii) ein Hydrocarbylol, ausgenommen Polyole, die 2 oder mehr Hydroxylgruppen aufweisen,
unter solchen Bedingungen, daß sich ein Derivat ergibt, das im Durchschnitt mindestens 1,25 Mol gebundenes 2,2-Dihydrocarbyloxyethanal pro Mol Aminoverbindung und mindestens etwa 2,0 1,2,2-Trihydrocarbyloxyethylgruppen pro Molekül Derivat enthält.

18. Verfahren nach Anspruch 17, dadurch gekennzeichnet, daß in einem ersten Schritt (i) und (ii) unter Anwesenheit eines Basenkatalysators in Kontakt gebracht werden, um ein 1-Hydroxy-2,2-Dihydrocarbyloxyethylderivatzwischenprodukt zu erzeugen, das in einem zweiten Schritt unter aziden Bedingungen in Kontakt mit (iii) gebracht wird, um das N-1,2,2-Trihydrocarbyloxyethylderivat der Aminoverbindung zu erzeugen.

19. Verfahren nach Anspruch 17, dadurch gekennzeichnet, daß (i), (ii) und (iii) gleichzeitig in Kontakt gebracht werden unter Anwesenheit eines Säurekatalysators, um direkt das N-1,2,2-Trihydrocarbyloxyethylderivat der Aminoverbindung zu erzeugen.

20. Vernetzbare Zusammensetzung, umfassend:
(a) eine Vernetzerkomponente, umfassend ein N-1,2,2-Trihydrocarbyloxyethylderivat einer Aminoverbindung nach einem der Ansprüche 1 - 16; und
(b) eine Harzkomponente, umfassend eine Verbindung, die mindestens zwei Gruppen enthält, die in der Lage sind, mit den 1,2,2-Trihydrocarbyloxyethylgruppen aus (a) zu reagieren.

21. Substrat, das mit einem vernetzten Film beschichtet ist, der aus der vernetzbaren Zusammensetzung nach Anspruch 20 abgeleitet ist.

## Revendications

1. Dérivé N-1,2,2-trihydrocarbyloxyéthylé d'un composé aminé, comprenant le produit de réaction :
(i) d'un composé aminé comportant au moins deux groupes =NH, choisi parmi les amino-1,3,5-triazines, les glycoluriles et leurs oligomères,
(ii) d'un 2,2-dihydrocarboxyloxy éthanal, et
(iii) d'un hydrocarbylol, à l'exclusion des polyols comportant deux groupes hydroxyle ou plus ;
le produit de réaction contenant, en moyenne, au moins 1,25 mole de 2,2-dihydrocarbyloxy éthanal combiné par mole de composé aminé, et au moins environ 2,0 groupes 1,2,2-trihydrocarbyloxyéthyle par molécule de dérivé.

2. Dérivé N-1,2,2-trihydrocarbyloxyéthylé selon la revendication 1, caractérisé en ce que le composé aminé est choisi parmi les amino-1,3,5-triazines et les glycoluriles correspondants aux formules générales (IV) et (V) : 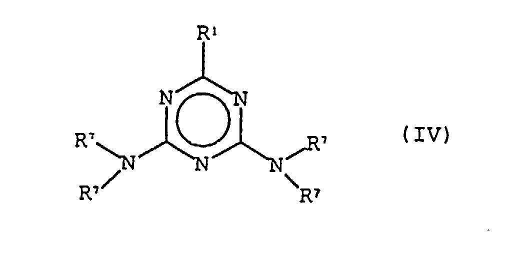 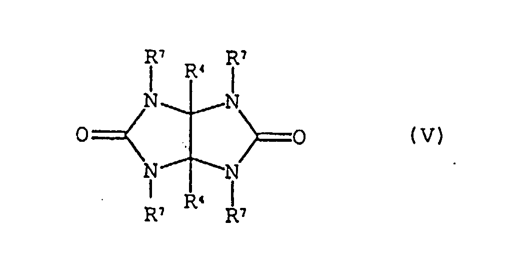
dans lesquelles R¹ est choisi parmi H, un groupe hydrocarbyle et -N(R⁷)₂,
chaque groupe R⁷ est indépendamment choisi parmi H et un groupe hydrocarbyle, à condition qu'au moins deux groupes R⁷ représentent H, et de préférence, que la totalité des groupes R⁷ représente H, et
chaque groupe R⁴ est indépendamment choisi parmi H et un groupe hydrocarbyle, de préférence, la totalité des groupes R⁴ représente H.

3. Dérivé de N-1,2,2-trihydrocarbyloxyéthylé selon la revendication 2, caractérisé en ce que le composé aminé correspond à la formule générale (IV), et tous les groupes R⁷ représentent H.

4. Dérivé N-1,2,2-trihydrocarbyloxyéthylé selon la revendication 3, caractérisé en ce que le dérivé est un dérivé de guanamine contenant, en moyenne, d'environ 1,5 à environ 2,0 moles de 2,2-dihydrocarbyloxy éthanal combiné par mole de guanamine.

5. Dérivé N-1,2,2-trihydrocarbyloxyéthylé selon la revendication 3, caractérisé en ce que le dérivé est un dérivé de mélamine contenant, en moyenne, d'environ 2,0 à environ 3,0 moles de 2,2-dihydrocarbyloxy éthanal combiné par mole de mélamine.

6. Dérivé N-1,2,2-trihydrocarbyloxyéthylé selon la revendication 2, caractérisé en ce que le composé aminé correspond à la formule générale (V), tous les groupes R⁴ représentent H, et tous les groupes R⁷ représentent H.

7. Dérivé N-1,2,2-trihydrocarbyloxyéthylé selon la revendication 6, caractérisé en ce que le dérivé est un dérivé de glycolurile contenant, en moyenne, d'environ 2,0 à environ 4,0 moles de 2,2-dihydrocarbyloxy éthanal combiné par mole de glycolurile.

8. Dérivé N-1,2,2-trihydrocarbyloxyéthylé selon la revendication 1, caractérisé en ce que le 2,2-dihydrocarbyloxy éthanal correspond à la formule générale (VI) : 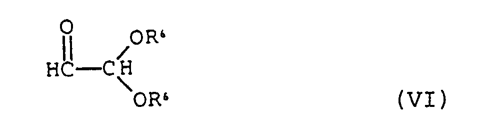 dans laquelle chaque groupe R⁶ représente indépendamment un groupe hydrocarbyle, ou ils forment ensemble un pont hydrocarbylène.

9. Dérivé N-1,2,2-trihydrocarbyloxyéthylé selon la revendication 8, caractérisé en ce que chaque groupe R⁶ représente indépendamment un groupe alkyle de 1 à 8 atomes de carbone ou un groupe alcényle de 1 à 8 atomes de carbone, ou les deux groupes R⁶ forment ensemble un pont alkylène de 1 à 8 atomes de carbone.

10. Dérivé N-1,2,2-trihydrocarbyloxyéthylé selon la revendication 2, caractérisé en ce que le 2,2-dihydrocarbyloxy éthanal correspond à la formule générale (VI) : 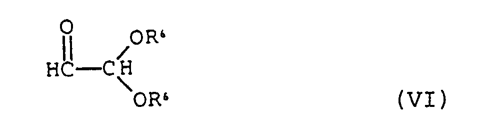 dans laquelle chaque groupe R⁶ représente indépendamment un groupe hydrocarbyle, ou ils forment ensemble un pont hydrocarbylène.

11. Dérivé N-1,2,2-trihydrocarbyloxyéthylé selon la revendication 1, caractérisé en ce que l'hydrocarbylol est un composé à groupe hydroxy comportant de 1 à 20 atomes de carbone.

12. Dérivé N-1,2,2-trihydrocarbyloxyéthylé selon la revendication 2, caractérisé en ce que l'hydrocarbylol est un composé à groupe hydroxy comportant de 1 à 20 atomes de carbone.

13. Dérivé N-1,2,2-trihydrocarbyloxyéthylé selon la revendication 10, caractérisé en ce que l'hydrocarbylol est un composé à groupe hydroxy comportant de 1 à 20 atomes de carbone.

14. Composé comprenant un noyau aminé comportant au moins deux groupes 1,2,2-trihydrocarbyloxyéthylé latéraux correspondants à la formule générale (I) ou (II) suivante : 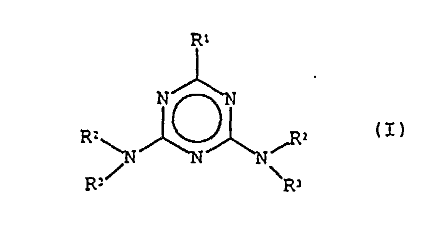 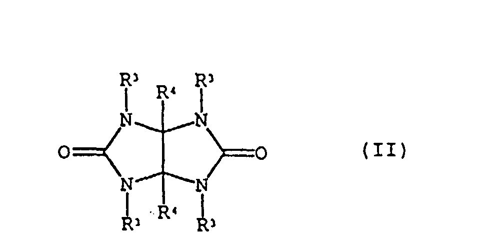 dans lesquelles
R¹ est choisi parmi H, un groupe hydrocarbyle et -N(R²)(R³) ;
chaque groupe R² est indépendamment choisi parmi H et un groupe hydrocarbyle ;
chaque groupe R³ est indépendamment choisi parmi H, un groupe hydrocarbyle et un groupe R ;
chaque groupe R⁴ est indépendamment choisi parmi H et un groupe hydrocarbyle ; et
chaque groupe R représente indépendamment un groupe de formule générale (III) : 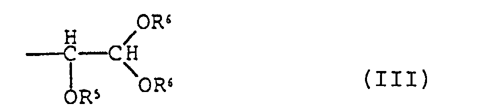
dans laquelle chaque groupe R⁵ est indépendamment choisi parmi H et un groupe hydrocarbyle, et
chaque groupe R⁶ représente indépendamment un groupe hydrocarbyle, ou ils forment ensemble un pont hydrocarbylène ;
à condition que, par molécule, au moins deux des groupes R³ représentent indépendamment un groupe R, et au moins deux groupes R⁵ représentent indépendamment un groupe hydrocarbyle.

15. Composé selon la revendication 14, caractérisé en ce que le composé correspond à la formule générale (I) ; chaque groupe R² représente H ; chaque groupe R³ représente un groupe R ; chaque groupe R⁵ est indépendamment choisi parmi H et un groupe alkyle comportant de 1 à 8 atomes de carbone, à condition qu'au moins deux des groupes R⁵ représentent un groupe alkyle comportant de 1 à 8 atomes de carbone ; et chaque groupe R⁶ représente indépendamment un groupe alkyle comportant de 1 à 8 atomes de carbone ou un groupe alcényle comportant de 1 à 8 atomes de carbone, ou les deux groupes R⁶ présents sur chaque groupe de formule (III) forment ensemble un pont alkylène comportant de 1 à 8 atomes de carbone.

16. Composé selon la revendication 14, caractérisé en ce que le composé correspond à la formule générale (II) ; chaque groupe R⁴ représente H ; chaque groupe R³ est choisi parmi H et un groupe R, à condition qu'au moins deux des groupes R³ représentent un groupe R ; chaque groupe R⁵ est choisi parmi H et un groupe alkyle comportant de 1 à 8 atomes de carbone, à condition qu'au moins deux des groupes R⁵ représentent un groupe alkyle comportant de 1 à 8 atomes de carbone ; et chaque groupe R⁶ représente indépendamment un groupe alkyle comportant de 1 à 8 atomes de carbone ou un groupe alcényle comportant de 1 à 8 atomes de carbone, ou les deux groupes R⁶ présents sur chaque groupe de formule (III) forment ensemble un pont alkylène comportant de 1 à 8 atomes de carbone.

17. Procédé de préparation d'un dérivé N-1,2,2-trihydrocarbyloxyéthylé d'un composé aminé selon l'une quelconque des revendications 1 à 13, caractérisé en ce que le procédé comprend l'étape de mise en contact :
(i) d'un composé aminé comportant au moins deux groupes =NH, choisi parmi les amino-1,3,5-triazines, les glycoluriles et leurs oligomères,
(ii) d'un 2,2-dihydrocarboxyloxy éthanal, et
(iii) d'un hydrocarbylol, à l'exclusion des polyols comportant deux groupes hydroxyle ou plus ;
dans des conditions appropriées pour former un dérivé contenant, en moyenne, au moins 1,25 mole de 2,2-dihydrocarbyloxy éthanal combiné par mole de composé aminé, et au moins environ 2,0 groupes 1,2,2-trihydrocarbyloxyéthyle par molécule de dérivé.

18. Procédé selon la revendication 17, caractérisé en ce que dans une première étape, les composants (i) et (ii) sont mis en contact en présente d'un catalyseur basique pour produire un dérivé 1-hydroxy-2,2-dihydrocarbyloxyéthylé intermédiaire qui, dans une deuxième étape, est mis en contact avec le composant (iii) dans des conditions acides pour produire le dérivé N-1,2,2-trihydrocarbyloxyéthylé du composé aminé.

19. Procédé selon la revendication 17, caractérisé en ce que les composants (i), (ii) et (iii), sont simultanément mis en contact en présence d'un catalyseur acide afin de produire directement le dérivé N-1,2,2-trihydrocarbyloxyéthylé du composé aminé.

20. Composition durcissable, comprenant :
(a) un composant réticulant contenant un dérivé N-1,2,2-trihydrocarbyloxyéthylé d'un composé aminé selon l'une quelconque des revendications 1 à 16 ; et
(b) un composant résineux comprenant un composé contenant au moins deux groupes capables de réagir avec les groupes 1,2,2-trihydrocarbyloxyéthyle du composant (a).

21. Substrat revêtu d'un film réticulé dérivé de la composition durcissable de la revendication 20.
